# EUROPÄISCHE PATENTANMELDUNG

(11) **EP 0 872 257 A2**
(43) Veröffentlichungstag der Anmeldung: **21.10.1998**
(21) Anmeldenummer: 97106195.7
(22) Anmeldetag: 15.04.1997
(51) Int. Cl.: A61M 25/10

(54) **Katheter**

(71) Anmelder: Schneider (Europe) GmbH, 8180 Bülach (CH)
(72) Erfinder: Hofmann, Eugen, CH 8064 Zürich (CH)
(74) Vertreter: Schwepfinger, Karl-Heinz, Dipl.-Ing.

(57) **Zusammenfassung**

Ein Katheter (10) zum Behandeln eines Abschnitts eines eine Körperflüssigkeit führenden Gefäßes ist mit zwei an seinem distalen Endabschnitt (14) angebrachten Ballons (26, 28) zur Erzielung einer Abdichtung vor bzw. hinter dem zu behandelnden Abschnitt versehen. Im Katheter (10) ist wenigstens ein Lumen (15, 30) zur Zuführung eines Druckmittels zu den Ballons (26, 28), ein weiteres, zu wenigstens einer Öffnung (38) in der Katheterwand zwischen den beiden Ballons (26, 28) führendes Lumen (17, 36) zum Zuführen eines Behandlungsmittels und ein Lumen (40) zum Aufnehmen eines Führungsdrahtes vorgesehen. Das zum Aufnehmen eines Führungsdrahtes bestimmte Lumen (40) ist nur in den Endabschnitt (14) des Katheters (10) ausgebildet und erstreckt sich zwischen einer in der Katheterwand auf der proximalen Seite des proximalen Ballons (26) angebrachten Öffnung (42) und einer am distalen Ende des Endabschnitts (14) liegenden Öffnung (44).

## Beschreibung

Die Erfindung bezieht sich auf einen Katheter gemäß dem Oberbegriff des Patentanspruchs 1.

Ein solcher Katheter ist aus der EP-B-0 080 436 bekannt. Mit Hilfe der beiden Ballons kann in einem eine Körperflüssigkeit führenden Gefäß, beispielsweise ein Blutgefäß, ein Abschnitt mit Hilfe eines Mittels behandelt werden, das beispielsweise die Bildung einer neuen Verengung in dem Abschnitt verhindern soll. Während dieser Behandlung dichten die Ballons in aufgeblasenem Zustand den zu behandelnden Abschnitt nach beiden Seiten hin ab, so daß das Behandlungsmittel nicht mit der sonst durch das Gefäß transportierten Körperflüssigkeit in Kontakt kommt. Das Mittel kann nach der Behandlung durch das Lumen abgeleitet werden, über das es zugeführt worden ist.

Der bekannte Katheter wird über einen Führungsdraht in das Gefäß eingebracht, der in einem Lumen geführt wird, das sich über die gesamte Katheterlänge erstreckt. Aufgrund dieser Ausgestaltung des Katheters ist ein schnelles Auswechseln des Katheters gegen einen anderen nicht möglich, da hier zuerst am proximalen Ende des Führungsdrahtes eine Verlängerung angebracht werden muß, die es ermöglicht, den Führungsdraht beim Katheterwechsel an Ort und Stelle zu halten. Außerdem beeinträchtigt die große Länge, in der sich der Führungsdraht im Katheter befindet, die leichte Verschiebbarkeit des Katheters.

Aus der EP-B-0 203 945 ist zwar bereits ein Dilatationskatheter bekannt, bei dem der Führungsdraht nur im Bereich des distalen Endes des Katheters in einem Katheterlumen verläuft, so daß ein Katheterwechsel schnell ohne das Erfordernis einer Verlängerung des Führungsdrahtes durchgeführt werden kann, jedoch weist dieser Katheter an seinem distalen Ende nur einen einzigen Ballon auf, der zur Dilatation von Gefäßverengungen verwendet werden kann. Eine Behandlung eines Gefäßabschnittes mit einem speziell dafür vorgesehenen Mittel kann mit einem solchen Katheter nicht vorgenommen werden.

Der Erfindung liegt die Aufgabe zugrunde, einen Katheter der eingangs angegebenen Art so auszugestalten, daß er auf seinem Führungsdraht leicht verschoben werden kann und trotzdem ein einfaches und schnelles Auswechseln gestattet.

Erfindungsgemäß wird diese Aufgabe mit den im kennzeichnenden Teil des Patentanspruchs 1 angegebenen Merkmalen gelöst.

Aufgrund der erfindungsgemäßen Ausgestaltung des Katheters verläuft der Führungsdraht nur innerhalb des distalen Endabschnitts im Katheter, so daß ein Verschieben des Katheters ohne Überwindung größerer Reibungskräfte durchgeführt werden kann. Da der distale Endabschnitt nur ein kurzer Abschnitt des Katheters ist, läßt sich der Katheter vollständig längs des Führungsdrahtes aus dem Gefäß herausziehen. Der Führungsdraht kann dadurch sowohl zwischen der Punktionsöffnung und der Katheterspitze als auch proximal von der proximalen Öffnung des Führungsdrahtlumens angefaßt werden. Der Katheter kann dann sicher und schnell gegen einen anderen Katheter ausgetauscht werden, ohne daß eine Verlängerung des Führungsdrahtes vorgenommen werden muß, weil seine Lage im Gefäß bei diesem Auswechselvorgang unverändert bleibt. Ein weiterer Vorteil des Katheters besteht darin, daß infolge der kurzen Länge des Führungsdrahtlumens der Querschnitt des Katheters im gesamten proximalen Abschnitt reduziert ist und somit ein größerer freier Querschnitt für einen Kontrastmittelfluß um den dünnen Katheterschaft herum innerhalb des Führungskatheters vorhanden ist.

Die im Patentanspruch 2 gekennzeichnete vorteilhafte Weiterbildung ermöglicht eine individuelle Zuführung des Druckmittels zu den Ballons, was bei gewissen Behandlungsfällen vorteilhaft oder sogar erforderlich sein kann, insbesondere, wenn mittels eines Ballons (z.B. des distalen) die Engstelle im Gefäß zuerst aufgeweitet und dann erst zur weiteren Behandlung nach beiden Seiten hin abgedichtet werden soll.

Ein Ausführungsbeispiel wird nur unter Bezugnahme auf die Zeichnungen näher erläutert. Es zeigen:
- Figur 1 eine schematische Ansicht des erfindungsgemäßen Katheters,
- Figur 2 einen Schnitt längs der Linie 2-2 von Figur 1,
- Figur 3 einen Schnitt längs der Linie 3-3 von Figur 1,
- Figur 4 einen Schnitt längs der Linie 2-2 eines dem Katheter von Figur 1 äußerlich gleichenden Katheters mit geänderter innerer Struktur,
- Figur 5 einen Schnitt längs der Linie 3-3 des Katheters mit geänderter innerer Struktur, und
- Figur 6 eine weitere Ausgestaltung der Katheterspitze.

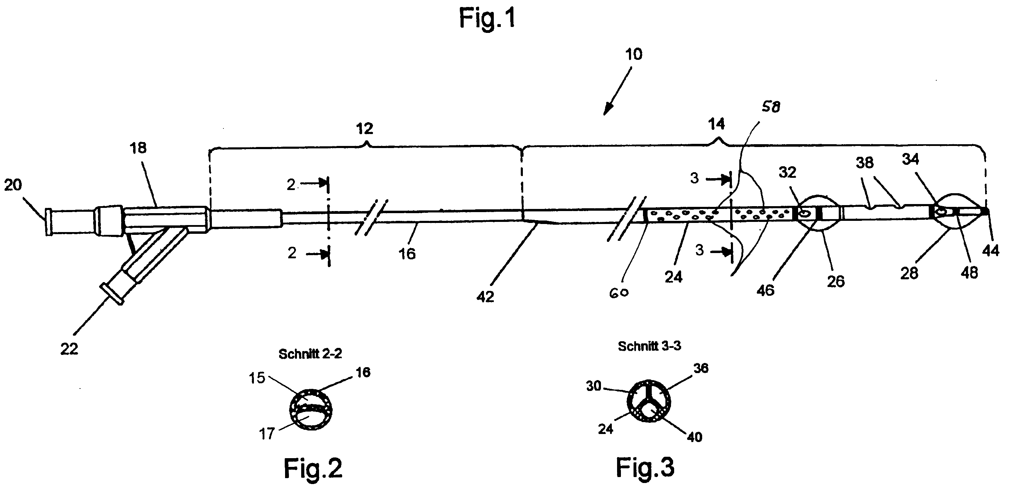

Der in Figur 1 dargestellte Katheter 10 ist aus einem proximalen Abschnitt 12 und einem distalen Endabschnitt 14 zusammengesetzt. Im proximalen Abschnitt 12 enthält der Katheter einen 2-lumigen Schlauch 16, der an seinem proximalen Ende mit einem Anschlußstück 18 versehen ist. Ein Eingang 20 des Anschlußstückes ermöglicht die Einführung eines Behandlungsmittels in eines der beiden Lumen des Schlauches 16, während über den zweiten Eingang 22 ein Druckmittel in das andere Lumen eingeführt werden kann. Die Schnittansicht von Figur 2 läßt die beiden Lumen 15, 17 des Schlauches 16 erkennen.

Der distale Endabschnitt 14 des Katheters 10 besteht aus einem 3-lumigen Schlauch 24, und er trägt zwei axial im Abstand voneinander liegende Ballons 26, 28, wie in Figur 1 zu erkennen ist. Diese zwei Ballons 26, 28 sind als Okklusionsballons ausgebildet. Das in der Schnittansicht von Figur 3 zu erkennende Lumen 30 setzt das der Druckmittelzufuhr dienende Lumen 15 des Katheterabschnittes 12 fort und mündet über Öffnungen 32, 34 in einen Ballon 26 bzw. 28. Das Lumen 36 setzt das der Zuführung des Behandlungsmittels dienende Lumen 17 im Katheterabschnitt 12 fort und führt zu zwei Öffnungen 38 in der Katheterwand im Bereich zwischen den beiden Ballons 26, 28. Das in der Schnittansicht von Figur 3 zu erkennende dritte Lumen 40 erstreckt sich im distalen Endabschnitt 14 zwischen einer Öffnung 42, die auf der proximalen Seite des proximalen Ballons 26 liegt, und einer Öffnung 44 an der Katheterspitze.

In der Praxis hat der Katheter 10 eine Gesamtlänge von beispielsweise 135 cm, während die Länge des distalen Endabschnitts, in dem der Schlauch 24 das Führungsdrahtlumen 40 aufweist, davon nur eine Länge von 22 cm einnimmt.

Bei der anschließenden Beschreibung einer Ausführungsform des beschriebenen Katheters wird angenommen, daß mittels des Katheters eine Engstelle in einem Gefäß beseitigt werden soll.

Bei der Durchführung dieser Behandlung wird in bekannter Weise zunächst ein Führungsdraht in dem Gefäß plaziert, in dem sich die zu behandelnde Engstelle befindet. Anschließend wird der Katheter auf das proximale Ende des Führungsdrahtes aufgeschoben, wobei das Aufschieben an der Öffnung 44 an der Katheterspitze beginnt. Aus der Öffnung 42 tritt der Führungsdraht aus dem Führungsdrahtlumen 40 wieder aus, worauf der Katheter dann längs des Führungsdrahtes soweit in das Gefäß eingeführt wird, bis sich die zu behandelnde Engstelle im Bereich zwischen den beiden Ballons 26 und 28 befindet. Die Lage der beiden Ballons kann dabei mit Hilfe von zwei innerhalb der Ballons angebrachten Goldmarkern 46, 48 auf dem Röntgenschirm beobachtet werden. Sobald sich die Ballons 26, 28 an der richtigen Position befinden, wird über den Eingang 22 des Anschlußstückes 18 Druckmittel zugeführt, so daß die beiden Ballons die Engstelle nach beiden Seiten hin abdichten. Über den Eingang 20 des Anschlußstückes 18 kann nun das Behandlungsmittel, beispielsweise ein Zytostatikum, eingeführt werden, das aus den Öffnungen 38 in den abgedichteten Zwischenraum zwischen den beiden Ballons ausströmt und auf das die aufgeweitete Engstelle umschließende Gewebe einwirkt. Das Behandlungsmittel kann über den Eingang 20 abgesaugt werden, worauf der Vorgang, falls erforderlich, auch wiederholt werden kann.

Sollte es sich als notwendig erweisen, den Katheter 10 während der Behandlung gegen einen anderen auszutauschen, weil unter Umständen Ballons mit anderen Größen oder gegebenenfalls auch in unterschiedlichem Abstand liegende Ballone benötigt werden, dann kann der Katheter 10 nach Ablassen des Druckmittels längs des Führungsdrahtes soweit zurückgezogen werden, bis sich der distale Endabschnitt 14 vollständig außerhalb des Gefäßes befindet. Der Führungsdraht bleibt dabei sowohl am proximalen Ende auf einer Seite des Endabschnitts 14 als auch am distalen Ende auf der anderen Seite des Endabschnitts 14 noch zugänglich, so daß er während des Austauschvorgangs sicher an Ort und Stelle festgehalten werden kann. Am Führungsdraht muß also keine Verlängerung angebracht werden, um den Katheteraustausch zu ermöglichen.

Der beschriebene Katheter 10 ermöglicht somit eine Behandlung von Gefäßabschnitten mit dem Ziel, eine Wiederverengung einer expandierten Engstelle zu verhindern, wobei gleichzeitig ein rasches und einfaches Austauschen des Katheters ermöglicht wird, falls sich dies während einer Behandlung als notwendig erweisen sollte.

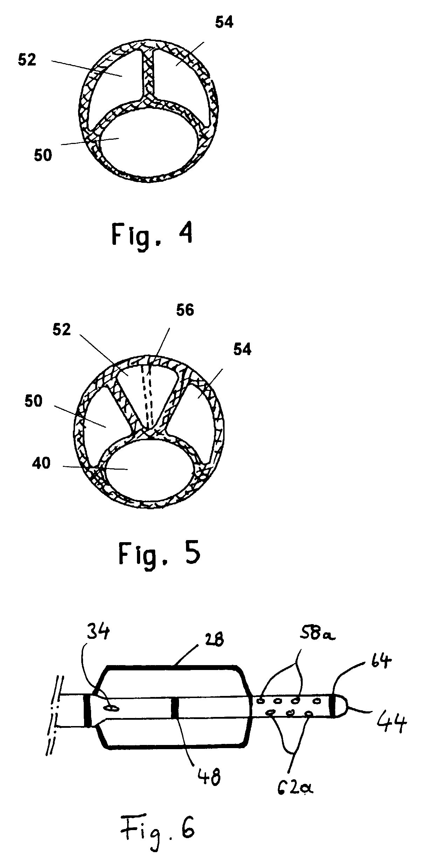

Zur dauerhaften Beseitigung einer Engstelle in einem Gefäß kann es auch erwünscht sein, die Engstelle zunächst mit Hilfe eines Ballons aufzuweiten, und die aufgeweitete Engstelle dann mit Hilfe der beiden Ballons nach beiden Seiten hin abzudichten und durch Zuführen eines Behandlungsmittels über das Lumen 17 in ihrer Rückbildung zu hemmen. Zur Ermöglichung dieser Behandlungsmethode wird der Behandlungskatheter von Figur 1 so abgewandelt, daß der distale Ballon 28 als Dilatationsballon ausgebildet wird und daß der Katheter in seinem proximalen Abschnitt 12 dreilumig ausgebildet ist und neben einem Behandlungslumen 50 zwei Lumen 52 und 54 aufweist, die zu jeweils einem der beiden Ballone 26 bzw. 28 führen. Ein Schnitt durch den proximalen Abschnitt 12 dieses Katheters ist in Figur 4 dargestellt. Aufgrund dieser inneren Struktur des Behandlungskatheters ist es möglich, den beiden Ballons das Druckmittel individuell zuzuführen.

Bei der Behandlung einer Engstelle wird der Katheter zunächst so weit vorgeschoben, daß sich der distale Ballon im Bereich der Engstelle befindet, so daß die Engstelle durch Zuführen des Druckmittels zu diesem Ballon aufgeweitet werden kann. Nach der Aufweitung wird der Katheter weitergeschoben, bis sich die aufgeweitete Engstelle zwischen den beiden Ballons befindet. Durch Zuführen von Druckmittel zu beiden Ballons wird die Engstelle dann zu beiden Seiten hin abgedichtet, und es kann das Behandlungsmittel über das Lumen 50 in den abgedichteten Bereich zwischen den beiden Ballons eingeführt werden.

Die Ausbildung des distalen Ballons als Dilatationsballon kann durch die Wahl eines Ballonmaterials erfolgen, das den speziellen Anforderungen eines Dilatationsballons hinsichtlich Festigkeit, Abmessungen, etc. genügt.

Figur 5 zeigt einen Schnitt längs der Linie 3-3 durch den distalen Abschnitt 14 des Katheters mit der geänderten inneren Struktur, wobei der Katheter in diesem distalen Abschnitt 14 vier Lumina aufweist, nämlich das übliche Führungsdrahtlumen 40 und die drei auch schon in Figur 4 zu erkennenden Lumen 50, 52, und 54.

Bei dem beschriebenen Behandlungskatheter ist in beiden Ausführungsformen eine Strömungsverbindung zwischen der proximalen Seite des proximalen Ballons 26 und der distalen Seite des distalen Ballons 28 vorhanden, wobei diese Verbindung durch das Führungsdrahtlumen 40 hergestellt wird. Der Führungsdraht füllt dieses Lumen 40 nämlich nicht vollständig aus, so daß dieses Führungsdrahtlumen in diesem Abschnitt des Katheters gleichzeitig die Funktion eines Perfusionslumens hat. Auf diese Weise wird verhindert, daß die in dem Gefäß strömende Flüssigkeit, bei der es sich beispielsweise um Blut handelt, wenn ein Blutgefäß behandelt wird, daher auch während der Behandlung mittels des Katheters weiterhin durch das Gefäß strömen kann. Falls die eingeschränkte Flüssigkeitsströmung durch das Führungsdrahtlumen 40 zu gering ist, kann zusätzlich ein eigenes Perfusionslumen vorgesehen werden, so daß der distale Abschnitt 14 des Behandlungskatheters dann insgesamt fünf Lumina aufweist. Diese abgeänderte Ausführung ist in Figur 5 dadurch veranschaulicht, daß im Lumen 52 eine gestrichelte Trennwand 56 angegeben ist. Auf diese Weise entsteht ein zusätzliches Lumen, das als Perfusionslumen verwendet werden kann.

Die Katheterwand weist auf der proximalen Seite des proximalen Ballons 26 Eintrittsöffnungen 58 auf, welche den Außenbereich des Katheterschaftes mit dem Perfusionslumen verbindet.

Alternativ oder in Ergänzung zu einem zusätzlichen Perfusionslumen kann auch der Führungsdraht bis zu einer röntgendichten Markierung 60 zurückgezogen werden, um den gesamten Querschnitt des Führungsdrahtlumens 40 für die Perfusion bereitzustellen. Die röntgendichte Markierung 60 ist dabei an einer Stelle angebracht, von der aus der Führungsdraht problemlos wieder durch das Drahtlumen vorgeschoben werden kann. In dieser Ausführungsform weist die Katheterwand ebenfalls seitliche Eintritts- und Austrittsöffnungen 62 auf, die das zur Perfusion benutzte Führungsdrahtlumen außerhalb des Behandlungsabschnittes mit den Flüssigkeit führenden Gefäßumgebungen des Katheterschaftes verbinden.

Zur Vergrößerung des Ausströmquerschnitts aus dem Perfusionslumen kann die Spitze des Katheters distal vom distalen Ballon 28 auch länglich ausgebildet sein und in der Katheterwand weitere, seitliche Austrittsöffnungen aus dem Perfusionslumen 58a bzw. aus dem Führungsdrahtlumen 62a aufweisen. Die Katheterspitze ist in diesem Fall mit einer röntgendichten Markierung versehen, um etwa bei einer Gefäßverzweigung kontrollieren zu können, ob die Spitze auch in den zu versorgenden Seitenast mündet.

## Patentansprüche

1. Katheter (10) zum Behandeln eines Abschnitts eines eine Körperflüssigkeit führenden Gefäßes, mit zwei axial im Abstand voneinander am distalen Endabschnitt (14) des Katheters (10) angebrachten Ballons (26, 28) zur Erzielung einer Abdichtung vor bzw. hinter dem zu behandelnden Abschnitt, wobei in dem Katheter (10) wenigstens ein Lumen (15, 30) zur Zuführung eines Druckmittels zu den Ballons (26, 28), ein weiteres, zu wenigstens einer Öffnung (38) in der Katheterwand zwischen den beiden Ballons (26, 28) führendes Lumen (17, 36) zum Zuführen eines Behandlungsmittels und ein Lumen (40) zum Aufnehmen eines Führungsdrahtes vorgesehen sind, dadurch gekennzeichnet, daß das Lumen (40) zum Aufnehmen eines Führungsdrahtes nur in dem Endabschnitt (14) des Katheters (10) ausgebildet ist und sich zwischen einer in der Katheterwand auf der proximalen Seite des proximalen Ballons (26) angebrachten Öffnung (42) und einer am distalen Ende des Endabschnitts (14) liegenden Öffnung (44) erstreckt.

2. Katheter nach Anspruch 1, dadurch gekennzeichnet, daß für jeden Ballon (26, 28) ein eigenes Lumen zum Zuführen des Druckmittels vorgesehen ist.

3. Katheter nach einem der vorhergehenden Ansprüche, dadurch gekennzeichnet, daß der distale Ballon (28) als Dilatationsballon ausgebildet ist, und daß der proximale Ballon (26) als Okklusionsballon ausgebildet ist.

4. Katheter nach einem der vorhergehenden Ansprüche, dadurch gekennzeichnet, daß sich durch den Endabschnitt (14) des Behandlungskatheters ein Perfusionslumen erstreckt, das auf der proximalen Seite des proximalen Ballons (26) zur Katheteraußenseite hin geöffnet ist und zu einer Öffnung an der Katheterspitze führt.

5. Katheter nach Anspruch 4, dadurch gekennzeichnet, daß das Führungsdrahtlumen und das Perfusionslumen über den distalen Ballon (28) hinaus verlängert sind und jeweils seitliche Austrittsöffnungen (58a, 62a) aufweisen.
